# EUROPÄISCHE PATENTSCHRIFT

(11) **EP 1 306 365 B1**
(45) Veröffentlichungstag und Bekanntmachung des Hinweises auf die Patenterteilung: **22.10.2008**
(21) Anmeldenummer: 02023640.2
(22) Anmeldetag: 21.10.2002
(51) Int. Cl.: C07C 209/48, C07C 211/09

(54) **Cobalt-Trägerkatalysatoren für Nitrilhydrierungen**
Cobalt supported catalysts for hydrogenation of nitriles
Catalyseurs supportés au cobalt pour l'hydrogénation de nitrile

(30) Priorität: 23.10.2001 DE 10152135
(43) Veröffentlichungstag der Anmeldung: 02.05.2003
(73) Patentinhaber: BASF SE, 67056 Ludwigshafen (DE)
(72) Erfinder: Ansmann, Andreas, Dr., 69168 Wiesloch (DE); Benisch, Christoph, Dr., 68165 Mannheim (DE)
(74) Vertreter: Isenbruck, Günter

(56) Entgegenhaltungen:
- EP-A- 0 424 738
- EP-A- 0 566 197
- DE-A- 1 518 118

## Beschreibung

Die Erfindung betrifft ein Verfahren zur Herstellung primärer Amine durch Hydrierung von Nitrilen in Anwesenheit eines Trägerkatalysators enthaltend Cobalt und gegebenenfalls zusätzlich Nickel. Des weiteren betrifft die Erfindung die Verwendung dieses Katalysators in einem Verfahren zur Herstellung primärer Amine durch Hydrierung von Nitrilen.

Bei der Hydrierung von Nitrilen werden, in Abhängigkeit von den Reaktionsbedingungen und dem verwendeten Katalysator, primäre, sekundäre oder tertiäre Amine gebildet, wobei im allgemeinen eine Mischung aller drei Amintypen erhalten wird. In der Vergangenheit wurde intensiv danach geforscht, die selektive Hydrierung zu einem Amintyp zu kontrollieren. Die Abtrennung von sekundären und tertiären Aminen von den beispielsweise gewünschten primären Aminen erfordert zusätzliche Apparaturen und zusätzliche Energie. Es wurde gefunden, daß eine erhöhte Selektivität zu primären Aminen dadurch erreicht werden kann, daß der Reaktionsmischung Ammoniak zugegeben wird. Bei der Hydrierung von Dinitrilen besteht das Problem, daß Kondensationsreaktionen auftreten können, die zu cyclischen Produkten oder Oligomeren führen.

Des weiteren wird die Selektivität der Hydrierung von Nitrilen durch die Natur des Katalysators beeinflußt. Die Natur des Katalysators beeinflußt des weiteren die zur Hydrierung der Nitrile notwendige Temperatur. So ist insbesondere bei Nitrilen, die unter drastischen Bedingungen wie hohen Temperaturen nicht stabil sind oder bei Hydrierung von Nitrilen, wobei z.B. die Einstellung eines bestimmten Isomerenverhältnisses gewünscht ist, die Hydrierung bei möglichst geringen Temperaturen unter schonenden Bedingungen wünschenswert. Aus dem Stand der Technik ist bekannt, daß als Katalysatoren für die Hydrierung von Nitrilen bei möglichst geringen Temperaturen und mit hoher Selektivität Raney-Nickel bzw. Raney-Cobalt am besten geeignet sind.

So betrifft US 4,721,811 ein kontinuierliches Verfahren zur Herstellung linearer Polyamine durch Hydrierung von Polynitrilen. Als Katalysator werden Raney-Cobalt-Granalien eingesetzt, die durch Laugung entsprechender Nuggets der Legierung erhältlich sind.

Auch US 5,869,653 betrifft ein Verfahren zur katalytischen Hydrierung von Nitrilen. Als Katalysator wird ein Raney-Cobalt-Katalysator eingesetzt, wobei die Hydrierung in Anwesenheit von katalytischen Mengen Lithiumhydroxid und Wasser erfolgt.

Die Verwendung von Raney-Katalysatoren und deren Herstellung bringt jedoch Nachteile mit sich. So sind Raney-Katalysatoren pyrophor, so daß sie nur unter Flüssigkeit oder unter Luftausschluß transportiert, gelagert und in die Reaktoren eingefüllt werden dürfen. Des weiteren ist die Herstellung der Raney-Katalysatoren sehr aufwendig, da zunächst eine Ausgangslegierung durch Aufschmelzen hergestellt werden muß, die danach mit erheblichen Mengen an konzentrierten Basen ausgelaugt wird. Im Anschluß daran erfolgt eine Waschung mit Wasser, wobei stark basische wässrige Abfälle erzeugt werden, die aus ökologischen Gründen aufwendig aufgearbeitet oder entsorgt werden müssen.

Somit besteht großes Interesse daran, die Raney-Katalysatoren durch andere, weniger problematisch zu handhabende Katalysatoren zu ersetzen.

Eine Möglichkeit ist der Einsatz von Trägerkatalysatoren. Gemäß dem Stand der Technik besitzen diese jedoch im allgemeinen niedrige Selektivitäten. Es sind jedoch Trägerkatalysatoren bekannt, worin eine Selektivitätserhöhung durch Dotierung oder durch die Verwendung von Co-Katalysatoren vorgeschlagen wird.

So betrifft EP-A 0 566 197 ein Verfahren zur Herstellung von primären Aminen durch Hydrierung von Mono- und/oder Dinitrilen mit Wasserstoff in Anwesenheit von Nickel- und/oder Cobaltkatalysatoren auf einem Trägermaterial, bevorzugt in Kombination mit mindestens einem festen, in dem Reaktionsmedium unlöslichen Cokatalysator, wobei der Katalysator und/oder der Cokatalysator im wesentlichen nicht sauer sind. Gemäß den Beispielen wurde festgestellt, daß durch die Zugabe von Alkalimetallen bzw. von Erdalkalimetallen eine Selektivitätserhöhung bei geringfügig verminderter Aktivität erfolgt. Diese Umsetzungen finden jedoch bei 130 bzw. 140°C statt, d.h. bei Bedingungen, die für thermisch labile Nitrile ungeeignet sind.

Die Verwendung eines Cokatalysators bedeutet einen zusätzlichen Aufwand, insbesondere bei der Verwendung von Alkali- oder Erdalkalimetallen.

EP-A 0 424 738 betrifft ein Verfahren zur Herstellung linearer Triaminoverbindungen durch Umsetzung von 1,3,6-Tricyanohexan an einem Katalysator, der Cobaltoxid sowie ein Oxid von Alkalimetallen, Erdalkalimetallen, seltenen Erden oder Scandium oder Yttrium enthält. Dieser Katalysator kann als Voll- oder Trägerkatalysator ausgebildet sein. Der Katalysator besitzt eine unzureichende Aktivität, was an den geringen Raum-Zeit-Ausbeuten bzw. den drastischen Hydrierbedingungen (insbesondere: Druck von 300 bar) erkennbar ist.

DE-A 1 518 118 betrifft ein Verfahren zur Hydrierung von Nitrilen, wobei Cobalt-Katalysatoren eingesetzt werden, die Magnesiumoxid enthalten. Dabei kann es sich um Voll- oder Trägerkatalysatoren handeln. Gemäß Beispiel 1 in DE-A 1 518 118 werden als aktive Katalysatoren solche eingesetzt, in denen 55% des Cobalts als Cobalt-Metall vorliegen. Gemäß den Beispielen erfolgt die Hydrierung bei Temperaturen von 100 bis 140°C. Somit ist dieses Verfahren ebenfalls zur Hydrierung thermisch labiler Nitrile ungeeignet.

WO 97/10052 betrifft ein Verfahren zur Hydrierung von Nitrilen, worin Hydrierkatalysatoren eingesetzt werden, die mindestens ein zweiwertiges Metall in zum Teil reduzierten Zustand (bevorzugt Ni oder Co) und mindestens ein Dotiermetall aus der Gruppe Cr, Mo, Fe, Mn, Ti, V, Ga, In, Bi oder seltenen Erden in oxidischer Form enthalten. Des weiteren betrifft WO 97/10052 die Katalysatoren selbst. Bei den in WO 97/10052 offenbarten Katalysatoren handelt es sich um Vollkatalysatoren, die zum überwiegenden Teil aus dem Aktivmetall bestehen und daher sehr teuer sind.

Die Aufgabe der vorliegenden Erfindung ist es daher, ein Verfahren zur Herstellung primärer Amine durch Hydrierung von Nitrilen bereitzustellen, worin Katalysatoren eingesetzt werden, die problemlos zu handhaben sind, preiswerter sind als Vollkatalysatoren, hohe Aktivitäten und Selektivitäten für die Hydrierung von Nitrilen aufweisen und sowohl als Pulver im Suspensionsverfahren als auch als Formkörper in Festbettverfahren einsetzbar sind. Des weiteren sollen diese Katalysatoren ihre hohe Aktivität und Selektivität bezüglich primärer Amine bereits bei niedrigen Temperaturen aufweisen, so daß das Verfahren zur Hydrierung thermisch labiler Nitrile geeignet ist.

Die Lösung der Aufgabe geht aus von einem Verfahren zur Herstellung primärer Amine durch Hydrierung von Nitrilen in Anwesenheit eines Katalysators enthaltend Cobalt und gegebenenfalls zusätzlich Nickel sowie mindestens ein weiteres Dotiermetall auf einem teilchenförmigen Trägermaterial.

Das erfindungsgemäße Verfahren ist dann **dadurch gekennzeichnet, daß** das Cobalt und gegebenenfalls Nickel eine mittlere Teilchengröße von 3 bis 30 nm (Nanometer) im aktiven Katalysator aufweisen.

Bevorzugt beträgt die mittlere Teilchengröße im aktiven Katalysator 3 bis 20 nm, besonders bevorzugt 3 bis 15 nm, ganz besonders bevorzugt 3 bis 10 nm. Die mittlere Teilchengröße wurde durch Röntgenbeugung (Diffraktometer D5000, Fa. Siemens, Auswertesoftware TOPAS) ermittelt. Dieser erfindungsgemäß eingesetzte Katalysator weist aufgrund seiner großen aktiven Oberfläche und seiner kleinen Kristallite hohe Aktivitäten auf und zeichnet sich durch eine lange Lebensdauer aus. Dabei sind für das Erreichen der hohen Aktivitäten bereits geringe Mengen der katalytisch aktiven Spezies ausreichend, wodurch kostengünstige Katalysatoren erhältlich sind.

Diese erfindungsgemäß eingesetzten Katalysatoren werden nach einem Verfahren mit folgenden Schritten hergestellt:
a) gemeinsame Fällung von Cobalt und gegebenenfalls zusätzlich Nickel sowie mindestens einem weiteren Dotiermittel aus einer Lösung, die entsprechende Salze der genannten Metalle enthält, auf ein teilchenförmiges Trägermaterial,
b) anschließende Trocknung und/oder Calcinierung,
c) gegebenenfalls Verformung, und
d) Reduktion.

### a) Gemeinsame Fällung von Cobalt und gegebenenfalls zusätzlich Nickel sowie mindestens einem weiteren Dotiermetall

Die erfindungsgemäß eingesetzten Katalysatoren werden über eine gemeinsame Fällung (Mischfällung, Cofällung) ihrer Komponenten auf ein teilchenförmiges Trägermaterial hergestellt. Dazu wird im allgemeinen eine die Katalysatorkomponenten enthaltende, wässrige Salzlösung in der Wärme bei Temperaturen von 20 bis 100°C, bevorzugt 40 bis 80°C unter Rühren solange mit einer wässrigen Mineralbase, bevorzugt einer Alkalimetallbase, z.B. Natriumcarbonat, Natriumhydroxid, Kaliumcarbonat, Kaliumhydroxid oder Ammoniumcarbonat versetzt, bis die Fällung im wesentlichen vollständig ist. An Stelle einer Mineralbase kann auch eine organische Carbonsäure verwendet werden, die unlösliche Metallsalze bildet. Ein Beispiel für eine solche organische Carbonsäure ist Oxalsäure.

Die Art der verwendeten Metallsalze ist im allgemeinen nicht kritisch. Bei der gemeinsamen Fällung ist eine gute Wasserlöslichkeit der eingesetzten Salze wesentlich, so daß ein Kriterium betreffend die Art der verwendeten Metallsalze ihre zur Herstellung der verhältnismäßig stark konzentrierten Salzlösungen erforderliche gute Wasserlöslichkeit ist. Dabei ist wesentlich, daß bei der Auswahl der Salze der einzelnen Komponenten nur Salze mit solchen Anionen gewählt werden, die nicht zu Störungen, beispielsweise durch unerwünschte Fällungen oder durch Komplexierung, führen. Geeignete Salze sind im allgemeinen die entsprechenden Nitrate, Acetate, Formiate, Chloride, Sulfate und Oxalate. Die Konzentration der Salzlösungen beträgt im allgemeinen 5 bis 100%, bevorzugt 10 bis 90%, besonders bevorzugt 25 bis 75 % der maximalen Löslichkeit des Salzes/der Salze in Wasser.

Die gemeinsame Fällung kann nach beliebigen, dem Fachmann bekannten Verfahren durchgeführt werden. In der Regel ist es besonders vorteilhaft, während der gemeinsamen Fällung konstante Bedingungen einzustellen, um möglichst homogenes Fällgut zu erhalten. In einer Ausführungsform legt man daher die leicht basische Lösung der Mineralbase bei einem gewünschten pH-Wert von im allgemeinen 4 bis 8, bevorzugt 5 bis 7, besonders bevorzugt 5 bis 6 vor und gibt anschließend bevorzugt kontinuierlich die Lösungen der entspechenden Metallsalze (des Cobalts, gegebenenfalls des Nickels sowie von weiteren Dotiermetallen), das aufgeschlämmte Trägermaterial und weitere Mineralbase bei Konstanthaltung des pH-Wertes zu. Es ist jedoch bevorzugt, das Trägermaterial bei dem gewünschten pH-Wert von im allgemeinen 4 bis 8, bevorzugt 5 bis 7, besonders bevorzugt 5 bis 6 vorzulegen und anschließend die einzelnen Metallsalzlösungen des Cobalts, gegebenenfalls des Nickels und von Dotiermetallen zuzusetzen. Schließlich ist es auch möglich, das Trägermaterial zusammen mit den genannten Metallsalzen vorzulegen und anschließend die Mineralbase bis zur vollständigen Fällung zuzusetzen.

Im allgemeinen wird der Fällvorgang bei Temperaturen von 20 bis 100°C, bevorzugt 40 bis 90°C, besonders bevorzugt 50 bis 80°C durchgeführt. Der pH-Wert liegt in Abhängigkeit von den verwendeten Metallsalzen zwischen 4 und 7, bevorzugt zwischen 5 und 6.

In einer bevorzugten Ausführungsform wird die Reaktionsmischung im Anschluß an die Fällung noch eine gewisse Zeit, im allgemeinen 10 bis 90 min., bevorzugt 30 bis 60 min. bei der genannten Temperatur gerührt, gegebenenfalls unter Einblasen von Luft. Dadurch wird ein Nachaltern der Mischung und somit eine verbesserte Filtrierbarkeit erreicht. Schließlich wird bevorzugt der pH-Wert durch die Zugabe von weiterer Mineralbase auf einen Wert zwischen 7 und 9, bevorzugt zwischen 7,5 und 8,5 angehoben, um möglichst die Vollständigkeit der Fällung zu erreichen.

Im Anschluß an das Fällungsverfahren werden die erhaltenen Niederschläge abfiltriert und mit Wasser anionenfrei gewaschen. Das genaue Vorgehen ist dem Fachmann bekannt.

### b) Trockung und/oder Calcinierung

Nachdem die abfiltrierten Niederschläge mit Wasser anionenfrei gewaschen wurden, erfolgt die Trocknung und/oder Calcinierung der Niederschläge. Bevorzugt wird zunächst eine Trocknung und im Anschluß daran die Calcinierung durchgeführt. Die Trocknung der Niederschläge erfolgt im allgemeinen bei Temperaturen von 70 bis 150°C, bevorzugt 90 bis 130°C. Sie wird üblicherweise in einem Ofen oder in einem Sprühtrockner durchgeführt.

Um eine Zersetzung der je nach eingesetzten Anionen erhaltenen Carbonate, Hydroxide oder anderen Verbindungen zu erreichen, wird der gegebenenfalls getrocknete Feststoff calciniert. Die Calcinierung erfolgt bei Temperaturen von im allgemeinen 250 bis 600°C, vorzugsweise 300 bis 450°C. Geeignete Vorrichtungen zur Calcinierung sind beispielsweise Hordenöfen und Bandcalcinierer. Es ist sowohl bei der Trocknung als auch bei der Calcinierung möglich, ein Temperaturprogramm zu fahren, bei dem die Trocknung bzw. Calcinierung in mehreren Stufen bei unterschiedlichen Temperaturen erfolgt. Im allgemeinen dauert die Trocknung 2 bis 24 Stunden, bevorzugt 6 bis 15 Stunden. Die Calcinierung erfolgt im allgemeinen in einem Zeitraum von 1 bis 24 Stunden, bevorzugt 2 bis 10 Stunden, besonders bevorzugt 2 bis 5 Stunden.

### c) Gegebenenfalls Verformung des erhaltenen Pulvers

In einer bevorzugten Ausführungsform des erfindungsgemäßen Verfahrens werden die erfindungsgemäß eingesetzten Katalysatoren in einem Festbettverfahren eingesetzt. Für den Einsatz in einem Festbettverfahren ist eine Verformung des nach der Calcinierung erhaltenen Pulvers erforderlich. Diese Verformung kann nach dem Fachmann bekannten Verfahren beispielsweise durch Tablettierung oder Knetung und Extrusion erfolgen. Dazu werden Verformungshilfsmittel wie Tylose®, Walocel®, Kartoffelstärke, Polyvinylalkokhol, Milchsäure, Polyethylenoxid und Salpetersäure eingesetzt.

### d) Reduktion

Im Falle von Katalysatoren für den Einsatz im Festbettverfahren erfolgt im Anschluß an die Verformung die Reduktion der erhaltenen Katalysatorvorläufer. In einer weiteren Ausführungsform des erfindungsgemäßen Verfahrens werden die Katalysatoren als Pulver in einem Suspensionsverfahren eingesetzt. In diesem Falle erfolgt keine Verformung, sondern die Reduktion schließt sich direkt an die Calcinierung (Schritt b) an.

Die Reduktion wird mit Wasserstoff bei erhöhten Temperaturen von im allgemeinen 150 bis 800°C, besonders bevorzugt 200 bis 600°C durchgeführt. Bevorzugt führt man die Reduktion der erfindungsgemäß eingesetzten Cobalt-Katalysatoren in mehreren Stufen durch. Die erste Reduktionsstufe wird im allgemeinen bei 150 bis 450°C, bevorzugt 175 bis 400°C, besonders bevorzugt 200 bis 350°C durchlaufen, für 1 bis 24 Stunden, bevorzugt 1,5 bis 12 Stunden, besonders bevorzugt 2 bis 6 Stunden. Die zweite Reduktionsstufe erfolgt nach Aufheizen auf 300 bis 650°C, bevorzugt 350 bis 550°C, besonders bevorzugt 400 bis 500 °C. Diese dauert im allgemeinen 2 bis 48 Stunden, bevorzugt 4 bis 20 Stunden, besonders bevorzugt 6 bis 15 Stunden. An die zweite Reduktionsstufe kann sich gegebenenfalls eine dritte Reduktionsstufe anschließen, die in einem Bereich von 450 bis 800°C, bevorzugt 475 bis 700°C, besonders bevorzugt 500 bis 600 °C für einen Zeitraum von 0,5 bis 10 Stunden, bevorzugt 0,5 bis 8 Stunden, besonders bevorzugt 1 bis 6 Stunden erfolgt.

Die Reduktion wird im allgemeinen bei einem Druck von 0,1 bis 300 bar, bevorzugt 0,1 bis 100 bar, besonders bevorzugt 1 bis 10 bar durchgeführt. In einer ganz besonders bevorzugten Ausführungsform wird die Reduktion drucklos durchgeführt.

Ein wesentlicher Faktor für den Erhalt der erfindungsgemäß eingesetzten Katalysatoren ist das Verhältnis von Wasserstoff für die Reduktion zur Katalysatormenge. Die Reduktion wird daher bevorzugt bei einem Verhältnis von Wasserstoff zur Katalysatormenge von >1 Nm³/h x kg_{Kat.}, bevorzugt >2 Nm³/h x kg_{Kat.} durchgeführt. Besonders bevorzugt beträgt das Verhältnis von Wasserstoffmenge zu Katalysatormenge 1 bis 10 Nm³/h x kg_{Kat.}, ganz besonders bevorzugt von 1 bis 5 Nm³/h x kg_{Kat.}

Der Wasserpartialdruck im Wasserstoff, der im technischen Maßstab im allgemeinen im Kreis geführt wird, beträgt im allgemeinen ≤200 mbar, bevorzugt 0 bis 200 mbar, besonders bevorzugt 0 bis 100 mbar.

Mit Hilfe dieses erfindungsgemäßen Reduktionsschrittes können hochaktive Katalysatoren erhalten werden, die sich durch besonders kleine Cobalt- und gegebenenfalls Nickelkristallitgrößen sowie durch einen besonders hohen Reduktionsgrad auszeichnen. Die auf diese Weise erhaltenen Katalysatoren sind somit hochaktiv und besonders für das erfindungsgemäße Verfahren geeignet.

Die nach der Reduktion (d) erhaltenen Katalysatoren zeichnen sich dadurch aus, daß im allgemeinen mindestens 50%, bevorzugt mindestens 65%, besonders bevorzugt mindestens 70%, ganz besonders bevorzugt mindestens 75% des in dem Katalysator enthaltenen Cobalts bzw. gegebenenfalls Nickels in reduzierter Form vorliegen. Das bedeutet, daß die erfindungsgemäß eingesetzten Katalysatoren einen besonders hohen Anteil an aktiver Spezies enthalten und somit bereits bei niedrigen Temperaturen hoch aktiv sind. Das Cobalt bzw. gegebenenfalls das Nickel liegen in den erfindungsgemäß eingesetzten Katalysatoren entweder kubisch oder hexagonal vor.

Das in dem erfindungsgemäß eingesetzten Katalysator vorliegende Dotiermetall ist im allgemeinen ausgewählt aus Metallen der Gruppen III B, IV B, V B, VI B, VII B, II A, III, A und VI A und der Gruppe der Lanthaniden des Periodensystems der Elemente. Bevorzugt werden Dotiermetalle eingesetzt ausgewählt aus Metallen der Gruppen IIA, IIIB, IVB, IIIA, IVA und der Gruppe der Lanthaniden. Besonders bevorzugt werden Dotiermetalle ausgewählt aus Ti, Zr, La, Y, Gd, Ce, Si, Al, Ga und Mg eingesetzt. Mit Hilfe dieser Dotiermetalle können Katalysatoren erhalten werden, die neben einer hohen Aktivität hervorragende Selektivitäten in dem erfindungsgemäßen Verfahren aufweisen. Dabei ist es möglich, daß die erfindungsgemäß eingesetzten Katalysatoren neben dem mindestens einen Dotiermetall aus den genannten Gruppen mindestens ein weiteres Dotiermetall ausgewählt aus Silber, Gold und Ruthenium enthalten.

Als teilchenförmiges Trägermaterial ist im allgemeinen jedes beliebige inerte Trägermaterial geeignet. Beispielsweise können Trägermaterialien ausgewählt aus der Gruppe bestehend aus Siliciumdioxid, Aluminiumoxid, Titandioxid, Zirconiumdioxid, Siliciumdioxid/Aluminiumoxid, Alumosilicaten, Calciumoxid, Magnesiumoxid, Bims und Kohlenstoff oder Mischungen daraus eingsetzt werden. Bevorzugt werden Trägermaterialien ausgewählt aus Siliciumdioxid, Aluminiumoxid, Titandioxid, Zirconiumdioxid, Siliciumdioxid/Aluminiumoxid, Magnesiumoxid und Alumosilicat eingesetzt. Ganz besonders bevorzugt werden Siliciumdioxid und Alumosilicate sowie Aluminiumoxid und Siliciumdioxid/Aluminiumoxid und Magnesiumoxid als teilchenförmiges Trägermaterial eingesetzt.

Im allgemeinen wird das Dotiermetall in einer Menge von 1 bis 90 Gew.%, bevorzugt von 5 bis 70 Gew.%, besonders bevorzugt von 10 bis 50 Gew.% bezogen auf die eingesetzte Menge an Cobalt und gegebenenfalls Nickel eingesetzt. Das Massenverhältnis von Cobalt und gegebenenfalls Nickel zu der Summe aus Trägermaterial und mitgefälltem Dotiermetall in Form von Oxid liegt im allgemeinen im Bereich von 20 zu 80 bis 80 zu 20, bevorzugt 40 zu 60 bis 70 zu 30. Das Massenverhältnis von Cobalt zu Nickel liegt, für den Fall, daß Nickel in dem erfindungsgemäß eingesetzten Katalysator vorhanden ist, im Bereich von 90 zu 10 bis 99,9 zu 0,1, bevorzugt von 80:20 bis 99:1. Das Massenverhältnis von Trägermaterial zu dem mitgefällten Dotiermetall in Form von Oxid liegt im allgemeinen im Bereich von 98:2 bis 30:70, bevorzugt von 90:10 bis 50:50. Das Massenverhältnis von Cobalt und gegebenenfalls Nickel zu Silber, Gold oder Ruthenium liegt im allgemeinen Bereich von 90:10 bis 99,5:0,5.

Die so erhaltenen Katalysatoren sind hervorragend für die erfindungsgemäße Hydrierung von Nitrilen zu primären Aminen unter schonenden Bedingungen geeignet.

Die Hydrierung der Nitrile kann dabei auf unterschiedliche Weise durchgeführt werden, wobei geeignete Verfahrensbedingungen dem Fachmann bekannt sind. Die Hydrierung kann diskontinuierlich oder kontinuierlich durchgeführt werden, wobei eine kontinuierliche Fahrweise bevorzugt ist. Besonders bevorzugt wird die Hydrierung kontinuierlich in der flüssigen Phase durchgeführt. Die in dem erfindungsgemäßen Verfahren eingesetzten Katalysatoren sind insbesondere für eine kontinuierliche Hydrierung am Festbett oder in Suspension geeignet. Wird die Hydrierung am Festbett durchgeführt, so kann sie in der Rieselfahrweise (Durchströmung des Reaktors von oben nach unten) oder in der Sumpffahrweise (Durchströmung des Reaktors von unten nach oben) erfolgen. Geeignete Reaktoren für die Hydrierung am Festbett sind Rohrreaktoren. Besonders geeignete Ausführungsformen der Rohrreaktoren sind dem Fachmann bekannt.

Wird die Hydrierung in Suspension durchgeführt, so sind als Reaktoren insbesondere Rührkessel, Strahlschlaufenreaktoren oder Blasensäulen geeignet.

Es ist jedoch grundsätzlich auch möglich, das erfindunsgemäße Verfahren in einem diskontinuierlichen Betrieb durchzuführen. Dabei wird das Nitril oder eine Lösung des Nitrils gemeinsam mit dem Katalysator in einen Hochdruckautoklaven gegeben, anschließend Wasserstoff und gegebenenfalls Ammoniak aufgepreßt und das Reaktionsgemisch erhitzt. Nach Ende der Reaktion wird abgekühlt, der Katalysator abgetrennt und das Reaktionsgemisch fraktioniert destilliert.

Der zur Hydrierung verwendete Wasserstoff wird im allgemeinen in großem stöchiometrischen Überschuß verwendet. Er kann als Kreisgas in die Reaktion zurückgeführt werden. Der Wasserstoff kommt im allgemeinen technisch rein zum Einsatz. Beimengungen von Inertgasen, z.B. Stickstoff, stören jedoch den Reaktionsablauf nicht. Der Wasserstoffdruck beträgt im allgemeinen 0,5 bis 30 MPa, bevorzugt 2,0 bis 20 MPa, besonders bevorzugt 3,0 bis 10 MPa.

Die Temperatur in dem erfindungsgemäßen Verfahren beträgt im allgemeinen 25 bis 125 °C, bevorzugt 25 bis 100 °C, besonders bevorzugt 50 bis 100°C. Durch die vergleichsweise niedrigen Temperaturen, ist es möglich, mit dem erfindungsgemäßen Verfahren thermisch labile Nitrile zu hydrieren sowie die Einstellung bestimmter Isomerenverhältnisse, z.B. beim Isophorondiamin, zu ermöglichen. Auch bei solch geringen Temperaturen ist der erfindungsgemäß eingesetzte Katalysator aufgrund der kleinen mittleren Teilchengröße des Cobalts und gegebenenfalls Nickels und der dadurch erzeugten großen Oberfläche sehr gut.

Es ist möglich, in dem erfindungsgemäßen Verfahren zur Hydrierung von Nitrilen organische Lösungsmittel zuzusetzen. Dies ist dann sinnvoll, wenn das Nitril z.B. unter Normalbedingungen als Feststoff vorliegt. Bevorzugte organische Lösungsmittel sind aliphatische Alkohole, z.B. Methanol oder Ethanol, Amide, z.B. N-Methylpyrrolidon und Dimethylformamid, Ether, z.B. Dioxan oder Tetrahydrofuran sowie Ester. Besonders bevorzugt wird als Lösungsmittel Tetrahydrofuran eingesetzt.

Die Katalysatorbelastung liegt im allgemeinen im Bereich von 0,5 bis 20 mol Nitril/l_{Katalysator} x h, bevorzugt 2 bis 10 mol Nitril/l_{Katalysator} x h. Führt man eine Hydrierung von Di- oder Polynitrilen durch und strebt nur einen Teilumsatz an, können durch Veränderung der Verweilzeit der Umsatz und das Produktverhältnis eingestellt werden.

Es ist möglich, die Selektivität an primären Aminen durch die Zugabe von Ammoniak zu verbessern. Es werden jedoch bereits ohne die Zugabe von Ammoniak hervorragende Selektivitäten erzielt. Wenn Ammoniak verwendet wird, so liegt die Menge von Ammoniak im Bereich von im allgemeinen 6 bis 60 mol Ammoniak pro mol Nitril, bevorzugt 12 bis 36 mol Ammoniak pro mol Nitril.

Grundsätzlich lassen sich alle Nitrile nach dem erfindungsgemäßen Verfahren in primäre Amine überführen. Geeignete Nitrile sind sowohl Mononitrile als auch Dinitrile sowie Polynitrile mit mehr als zwei Nitrilgruppen. Bevorzugt werden Mononitrile oder Dinitrile in die entsprechenden Amine überführt. Das erfindungsgemäße Verfahren ist besonders geeignet zur Hydrierung von sogenannten Strecker-Nitrilen, die durch Anlagerung von Formaldehyd und Blausäure an nucleophile Zentren wie Amine erhältlich sind; von sogenannten Michael-Nitrilen, die durch die Anlagerung von Acrlynitril an nucleophile Zentren wie Amine zugänglich sind; von Iminonitrilen sowie von Dinitrilen, insbesondere α, ω-Dinitrilen.

Besonders bevorzugt werden daher in den erfindungsgemäßen Verfahren Nitrile der allgemeinen Formeln (I), (II), (III) oder (IV) eingesetzt:

R¹₃₋ₙX-A-CN (I)

worin die Symbole die folgende Bedeutung haben:
- X: O, S, N, P oder C, bevorzugt O, N oder C
- A: -(CR²R³⁾ₘ-, worin R² und R³ unabhängig voneinander substituiierte oder unsubstituiierte Aryl-, Alkyl- (verzweigt oder unverzweigt), Cykloalkylreste oder Wasserstoff bedeuten und m 0 bis 8, bevorzugt 1 bis 6, besonders bevorzugt 1 oder 2 ist, oder
eine C₂- bis C₈- Alkylenkette, wobei die Alkylenkette durch 1 bis 4, bevorzugt 1 bis 3, besonders bevorzugt 1 oder 2 Heteroatome, die nicht benachbart sind, unterbrochen ist, oder
Phenylen oder Cyclohexylen, gegebenenfalls substituiert mit substituierten oder unsubstituierten Aryl-, Alkyl- (verzweigt oder unvezweigt), Cykloalkylresten oder mit Heteroatome, insbesondere O oder N, enthaltendenden Resten;
- n: 2 (im Falle von X = O, S), 1 (im Falle von X = N, P) und 0 (im Falle von X = C);
- R¹: substituierte oder unsubstituierte Aryl-, Alkyl- (verzweigt oder unverzweigt), Cykloalkylreste oder Wasserstoff, wobei im Falle von m = 0 oder 1 R¹ auch unterschiedliche Reste aus der für R¹ genannten Gruppe sein können oder zwei Reste R¹ gemeinsam mit dem Heteroatom X (N, P, C) einen cyclischen Rest bilden können, der gegebenenfalls beliebig substituiert sein kann, wobei der Rest aromatisch oder cykloaliphatisch sein kann;

NC-A-CN (II)

worin A die obenstehende Bedeutung hat; 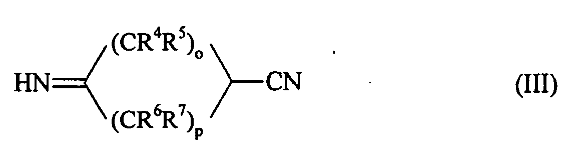 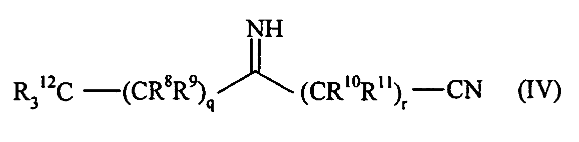 worin R⁴, R⁵, R⁶, R⁷, R⁸, R⁹, R¹⁰, R¹¹ und R¹² unabhängig voneinander substituierte oder unsubstituierte Aryl-, Alkyl- (verzweigt oder unverzweigt), Cykloalkylreste oder Wasserstoff bedeuten, und
- o, p, q, r: 0, 1, 2, 3, 4 oder 5 bedeuten.

Besonders bevorzugt werden Nitrile der allgemeinen Formeln (Ia) oder (IIa) eingesetzt:

R₃₋ₙX-(CR²R³)ₘ-CN (Ia)

worin
- X: O oder N bedeutet,
- m: 1 oder 2 ist, oder

NC-(CR²R³)ₘ-CN (IIa)
worin
R² und R³ unabhängig voneinander Alkylreste (verzweigt oder unverzweigt) oder Wasserstoff bedeuten und m 2 bis 4 ist.

Weiterhin bevorzugt sind Iminonitrile der allgemeinen Formel III oder IV.

In dem erfindungsgemäßen Verfahren bevorzugt eingesetzte Nitrile und die daraus entstehenden Amine sind in der folgenden Tabelle dargestellt.

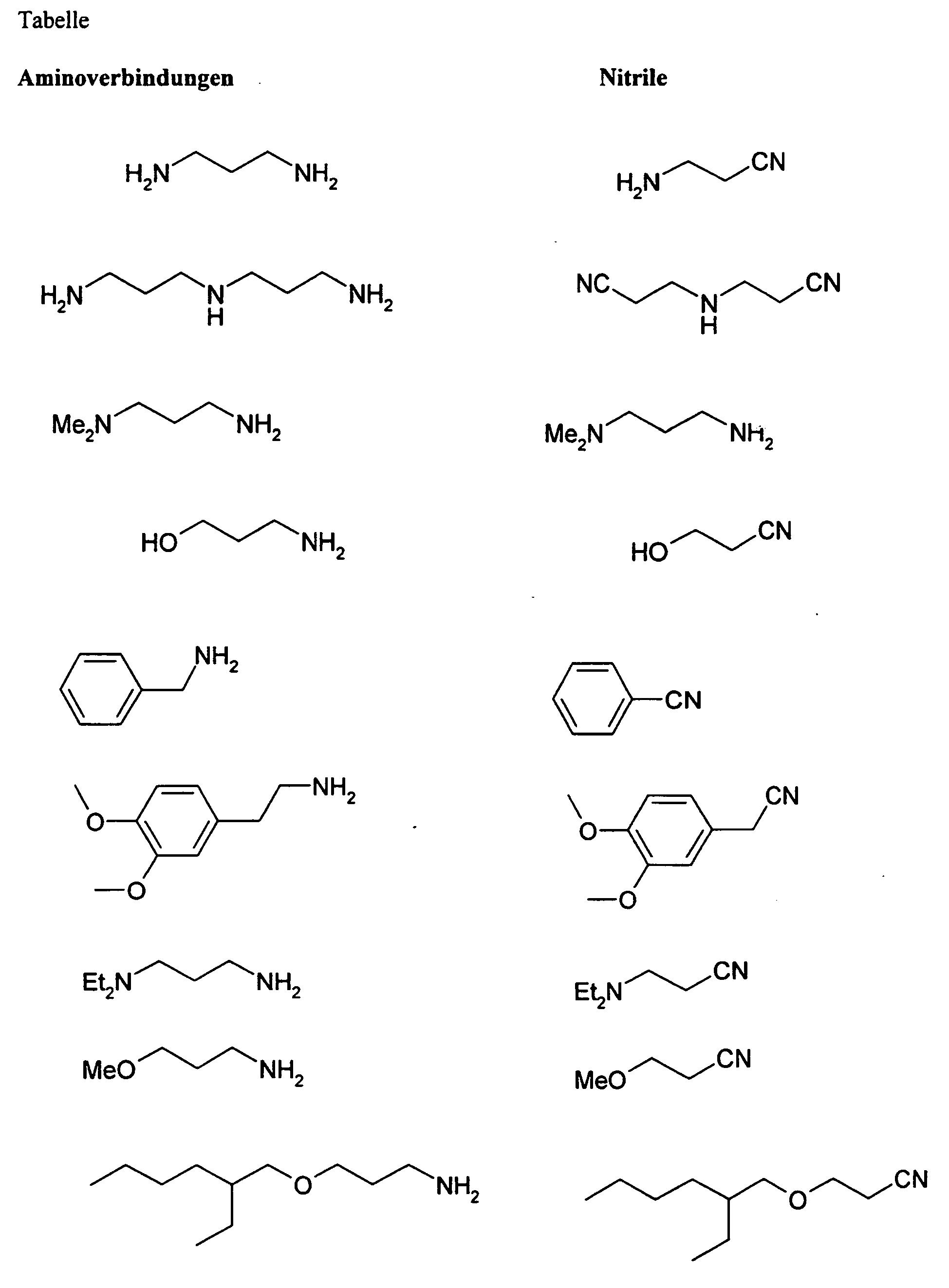

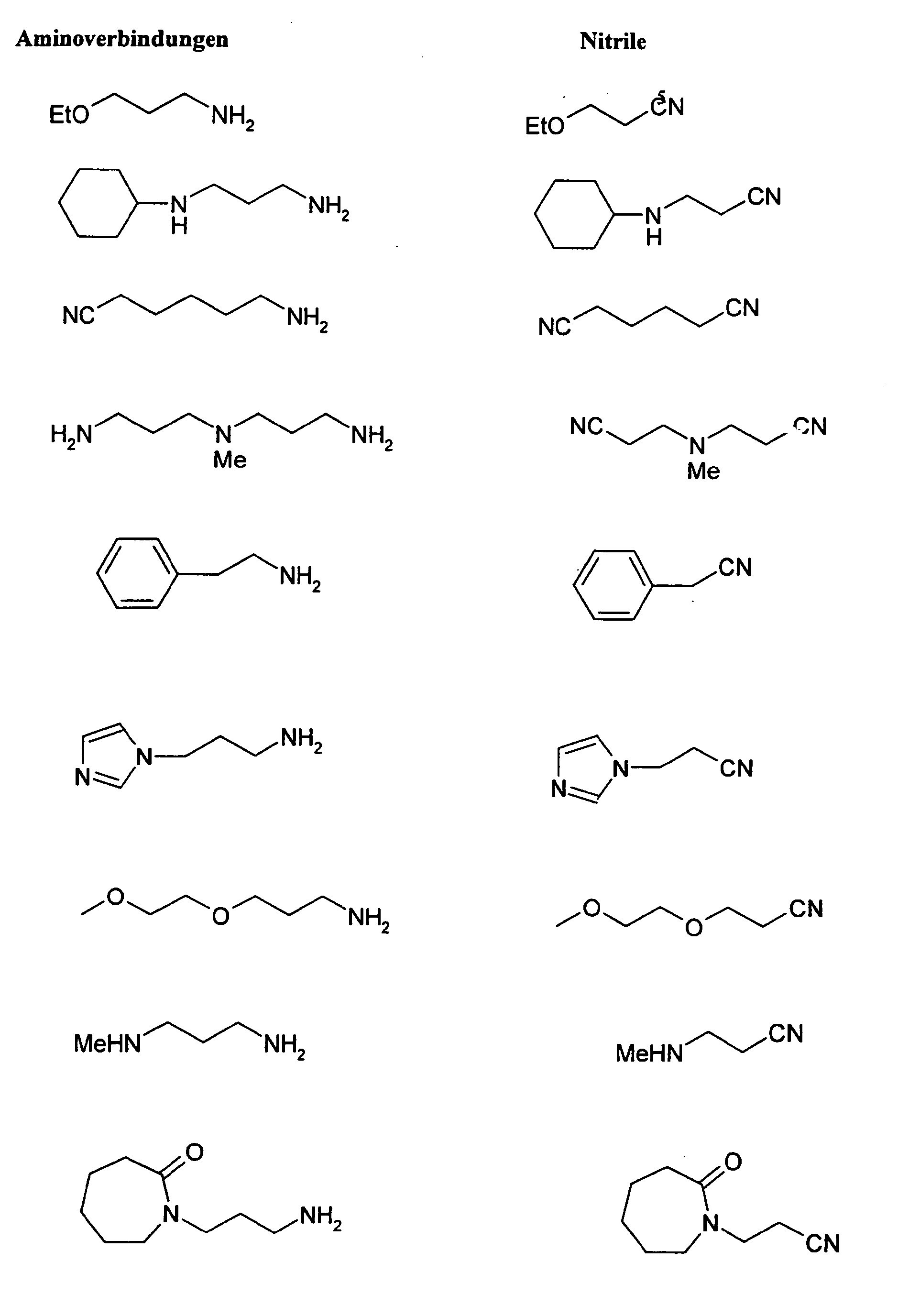

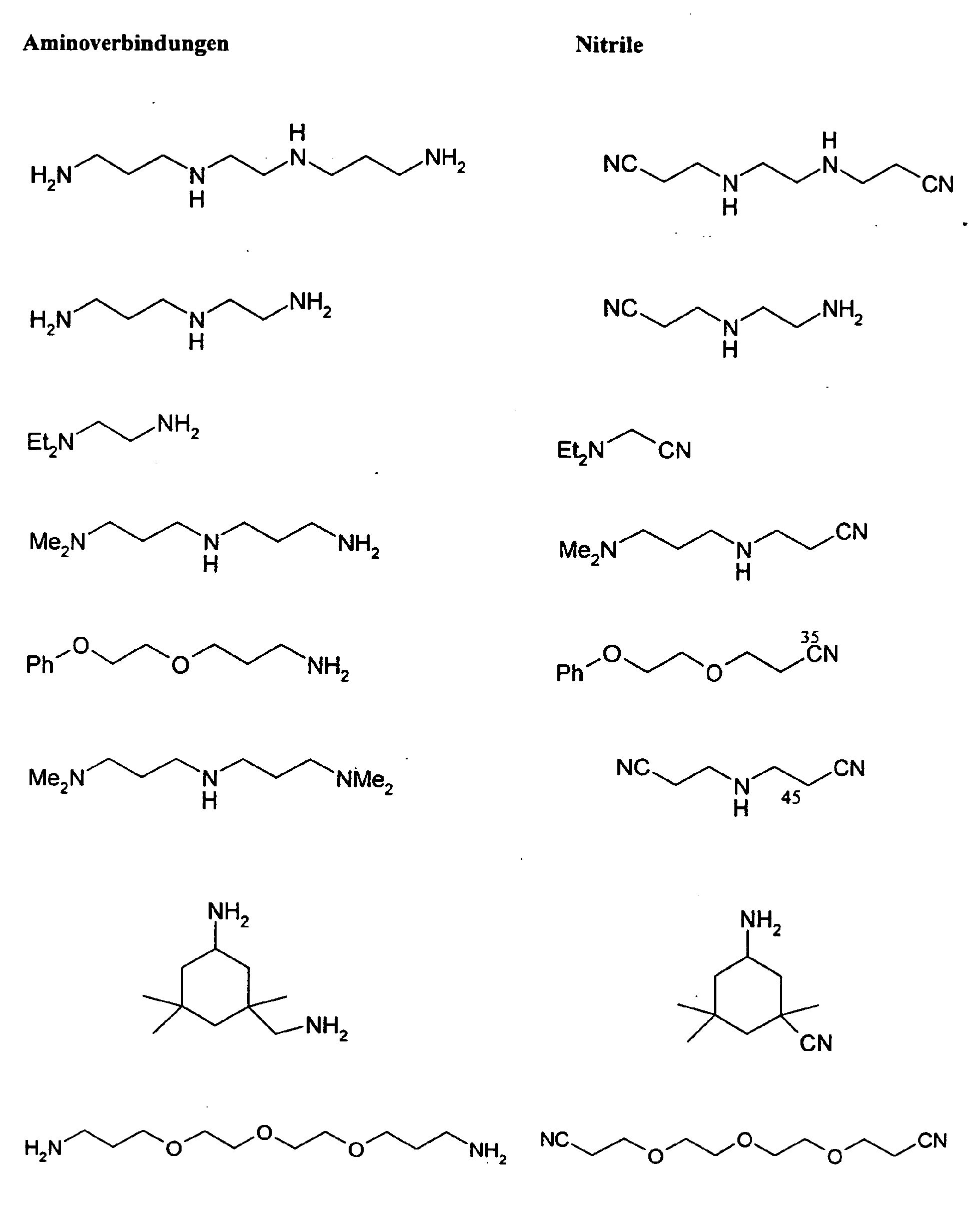

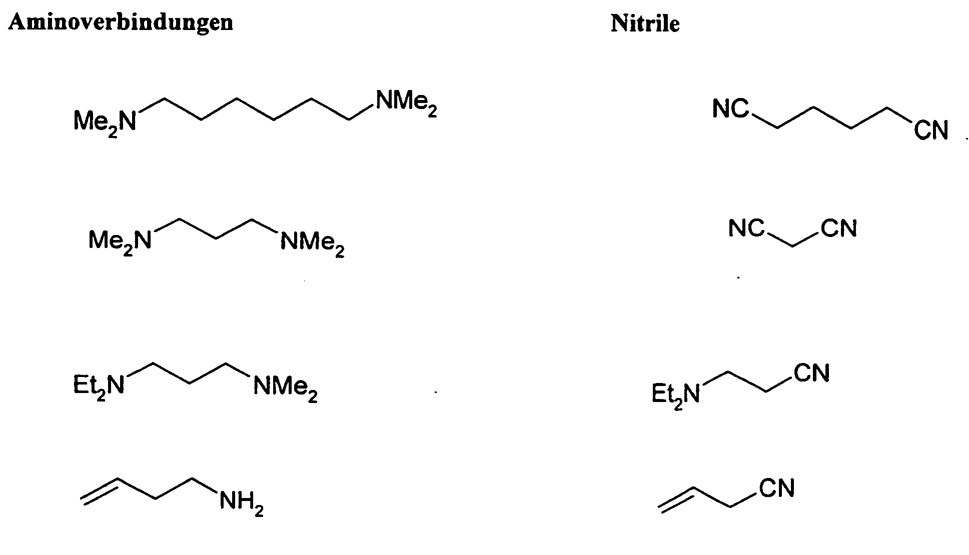

Ganz besonders bevorzugt werden Nitrile eingesetzt, ausgewählt aus Adipinsäurenitrilen, Imidodiacetonitril, Biscyanoethylpiperazin, Dimethylaminopropionitril und Isophoronnitrilimin.

Ein weiterer Gegenstand der vorliegenden Erfindung ist die Verwendung eines erfindungsgemäßen Katalysators in einem Verfahren zur Herstellung von primären Aminen durch die Hydrierung von Nitrilen. Bevorzugte Ausführungsformen des Katalysators sowie des Verfahrens zur Hydrierung und der geeigneten Nitrile sind bereits vorstehend genannt.

Die nachfolgenden Beispiele erläutern die Erfindung zusätzlich.

### Beispiele

### Beispiel 1

### Herstellung eines Festbettkatalysators

163,2 g eines Alumosilikatpulvers (Siral 40 von Condea) werden mit 2 1 voll entsalztem (VE-)Wasser aufgeschlämmt, mit einer Ultrathurax-Mühle fein dispergiert und in einem

Kolben vorgelegt. 1222,0 g Co(NO₃)₂*6 H₂O, 25,5 g Ni(NO₃)₂*6 H₂O und 625,0 g 20%-ige Zirkonacetatlösung werden in 4 1 VE-Wasser gelöst. Daneben wird eine 20%-ige Sodalösung (2885 g) bereitgestellt. Nun werden bei 90°C unter gutem Rühren die Sodalösung und die Metallsalzlösung gleichzeitig zugetropft unter Einhaltung des pH-Wertes von 5,6. Danach wird die Fällung für eine Stunde unter Durchleiten von Luft bei 90°C gerührt. Schließlich wird durch Zugabe weiterer Sodalösung der pH-Wert auf 7,5 angehoben. Nach Abnutschen der Fällung wird der Filterkuchen mit 100 1 VE-Wasser gewaschen und über Nacht bei 120°C getrocknet. Die so erhaltene Masse wird in einem Kneter kleingemahlen und dann mit 38,5 g Salpetersäure, 390 g VE-Wasser und 5 g Tylose (von Clariant) geknetet. Nachdem so eine plastische Masse erhalten werden konnte, wurde diese in einer Strangpresse mit einem Pressdruck von 75 bar zu 3 mm Strängen verformt. Diese wurden über Nacht bei 150°C getrocknet und anschließend für 4 h bei 400°C calciniert. 25 g dieser Stränge wurden mit 45 Nl/h Wasserstoff und 5 Nl/h Stickstoff nach folgendem Temperaturprogramm reduziert: Innerhalb einer Stunde aufheizen auf 300°C, 4 Stunden halten, innerhalb einer Stunde aufheizen auf 450°C, 10 Stunden halten, innerhalb einer Stunde aufheizen auf 540°C, 2 Stunden halten und unter Stickstoff abkühlen lassen.

Die Analyse der reduzierten Stränge durch Röntgenbeugung ergab, dass mehr als 80% des Cobalts in reduzierter Form vorliegen. Die mittlere Co-Kristallitgröße beträgt 8 nm. Der Co-Gehalt beträgt 49 Gew.-%, der Ni-Gehalt 1 Gew.-% der Gesamtmasse.

### Beispiel 2

### Herstellung eines Festbettkatalysators

125,0 g eines Siliziumdioxidpulvers (D11-10-40 von BASF) werden mit 2 1 VE-Wasser aufgeschlämmt, mit einer Ultrathurax-Mühle fein dispergiert und in einem Kolben vorgelegt. 1222,0 g Co(NO₃)₂*6H₂O, 25,5 g Ni(NO₃)₂*6H₂O und 625,0 g 20%-ige Zirkonacetatlösung werden in 4 1 VE-Wasser gelöst. Daneben wird eine 20%-ige Sodalösung (3134 g) bereitgestellt. Nun werden bei 90°C unter gutem Rühren die Sodalösung und die Metallsalzlösung gleichzeitig zugetropft unter Einhaltung des pH-Wertes von 5,6. Danach wird die Fällung für eine Stunde unter Durchleiten von Luft bei 90°C gerührt. Schließlich wird durch Zugabe weiterer Sodalösung der pH-Wert auf 7,5 angehoben. Nach Abnutschen der Fällung wird der Filterkuchen mit 100 1 VE-Wasser gewaschen und die Hälfte des Filterkuchens über Nacht bei 120°C, die andere Hälfte über Nacht bei 150°C getrocknet. Die so erhaltene Masse wird in einem Kneter kleingemahlen und dann mit 30 g VE-Wasser geknetet. Nachdem so eine plastische Masse erhalten werden konnte, wurde diese in einer Strangpresse mit einem Pressdruck von 70 bar zu 3 mm Strängen verformt. Diese wurden 16 Stunden lang bei 120°C getrocknet und anschließend für 4 h bei 400°C calciniert. 100 g dieser Stränge wurden mit 180 Nl/h Wasserstoff und 20 Nl/h Stickstoff nach folgendem Temperaturprogramm reduziert: Innerhalb einer Stunde aufheizen auf 300°C, 4 Stunden halten, innerhalb einer Stunde aufheizen auf 450°C, 10 Stunden halten, innerhalb einer Stunde aufheizen auf 540°C, 2 Stunden halten und unter Stickstoff abkühlen lassen.
Ein Teil der Stränge wurde analysiert, der größte Teil der Stränge wurde jedoch durch Überleiten einer Luft/Stickstoff-Mischung bei 50°C passiviert.
Die Analyse der reduzierten Stränge durch Röntgenbeugung ergab, dass mehr als 80% des Cobalts in reduzierter Form vorliegen. Die mittlere Co-Kristallitgröße beträgt 8 nm. Der Co-Gehalt beträgt 49%, der Ni-Gehalt 1% der Gesamtmasse.

### Beispiel 3

### Hydrierung von Adipinsäuredinitril im Festbett in Gegenwart des Katalysators aus Beispiel 1

In eine kontinuierlich betriebene Druckapparatur (Rohrreaktor, gerader Durchgang mit innenliegender Thermohülse) wurden 100 ml des Katalysators aus Beispiel 1 (= 89,1 g) eingebaut. Anschließend wurde der Reaktor mit Stickstoff inertisiert. Danach erfolgte die Reduktion des Katalysators durch Überleiten von 250 Nl/h Wasserstoff bei 70 bar nach folgendem Temperaturprogramm: Innerhalb von fünf Stunden aufheizen auf 300°C, 4 Stunden halten, innerhalb von drei Stunden aufheizen auf 450°C, 16 Stunden halten, innerhalb einer Stunde aufheizen auf 500°C, 3 Stunden halten und unter Wasserstoff abkühlen lassen. Nach Abkühlung der Anlage wurde mit 100 Nl/h Wasserstoff, 150 g/h Ammoniak und 50g/h Adipinsäure dinitril bei 70 bar und 65°C Eingangstemperatur angefahren. Die Feedbelastung beträgt somit 1,12 g Adipinsäure dinitril pro g Aktivmasse (Co+Ni). Nach 34 h Betriebszeit betrug der Umsatz an Adipinsäure dinitril 93,4%.

### Beispiel 4

### Hydrierung von Adipinsäuredinitril im Festbett in Gegenwart des Katalysators aus Beispiel 2

In eine kontinuierlich betriebene Druckapparatur (Rohrreaktor, gerader Durchgang mit innenliegender Thermohülse) wurden 100 ml des Katalysators aus Beispiel 2 (=86,3 g) in reduziert/passivierter Form eingebaut. Anschließend wurde der Reaktor mit Stickstoff inertisiert. Danach erfolgte die Aktivierung des passivierten Katalysators durch Überleiten von 250 Nl/h Wasserstoff bei 70 bar nach folgendem Temperaturprogramm: Innerhalb von sechs Stunden aufheizen auf 320°C, 40 Stunden halten, innerhalb von zwei Stunden aufheizen auf 450°C, 6 Stunden halten und unter Stickstoff abkühlen lassen. Nach Abkühlung der Anlage wurde mit 100 Nl/h Wasserstoff, 150 g/h Ammoniak und 50 g/h Adipinsäuredinitril bei 70 bar und 65°C Eingangstemperatur angefahren. Die Feedbelastung beträgt somit 1,16 g Adipinsäure dinitril pro g Aktivmasse (Co+Ni). Nach 22 h Betriebszeit betrug der Umsatz an Adipinsäure dinitril 88,1%.

### Vergleichsbeispiel 1

### Herstellung eines Festbettkatalysators nach Raney

Zu einer Mischung aus 900 g eines Pulvers aus einer Legierung aus 49,14 Gew.-% Aluminium, 49,2 Gew.-% Cobalt, 1,11 Gew.-% Chrom, 0,54 Gew.-% Nickel und 0,01 Gew.-% Eisen (ESSEX METALLURGICAL CO. LTD.), 80 g Polyvinylalkohol (Molekulargewicht 9000-18000 g/mol) und 8 g Tylose H2000 P (Clariant) wurden portionsweise 90 ml Wasser in einem Kneter hinzugefügt. Nach einer Knetzeit von drei Stunden wurde die plastische Masse bei einem Preßdruck von 14 MPa in einer Strangpresse zu 3 mm Strängen verformt. Die Stränge wurden für 16 h an Luft getrocknet, danach für 16 h zu 120°C getrocknet und anschließend 1 h bei 450°C, 1 h bei 750°C und 2 h bei 900°C calciniert. Die Stränge wurden danach in einem Backenbrecher zu Splitt mit der Kornfraktion 1,5 - 4 mm zerkleinert. 400 g dieser Stränge wurden bei 60°C zu 3000 g einer 20%igen Natronlauge hinzugefügt. Nach Abklingen der Wasserstoffentwicklung wurde auf 90°C aufgeheizt und für 3 h diese Temperatur gehalten. Der aktivierte Katalysator wurde mit Natronlauge und mit Wasser gewaschen und war dann für den Einbau in den Reaktor fertig. Der Gehalt an Cobalt betrug 45 Gew.-%, der Gehalt an Nickel 0,5 Gew.-%.

### Vergleichsbeispiel 2

### Hydrierung von Adipinsäuredinitril im Festbett in Gegenwart des Katalysators aus Vergleichsbeispiel 1

In eine kontinuierlich betriebene Druckapparatur (Rohrreaktor, gerader Durchgang mit innenliegender Thermohülse) wurde 200 ml des Katalysators aus Vergleichsbeispiel 1 (=236 g) eingebaut. Anschließend wurde der Reaktor mit Stickstoff inertisiert. Danach erfolgte eine Spülung des Reaktors mit 500 g/h Ethanol für fünf Stunden. Danach wurde mit 200 Nl/h Wasserstoff, 300 g/h Ammoniak und 100 g/h Adipinsäuredinitril bei 70 bar und 65°C Eingangstemperatur angefahren. Die Feedbelastung beträgt somit 0,93 g Adipinsäuredinitril pro g Aktivmasse (Co+Ni). Nach 21 h Betriebszeit betrug der Umsatz an Adipinsäuredinitril 63,0%.

Dieses Vergleichsbeispiel zeigt, dass die erfindungsgemäßen Co-Fällkatalysatoren eine höhere Aktivität als die Katalysatoren nach Raney aufweisen. Dabei bringen diese Fällkatalysatoren nicht die Nachteile des Umgangs mit sich wie die Katalysatoren nach Raney.

### Beispiel 5

### Herstellung eines Suspensionskatalysators

60,7 g eines Alumosilikatpulvers (Siral 40, Condea) werden mit 0,5 1 VE-Wasser aufgeschlämmt, mit einer Ultrathurax-Mühle fein dispergiert und in einem Kolben vorgelegt. 244,4 g Co(NO₃)₂*6H₂O, 5,1 g Ni(NO₃)₂*6 H₂O und 14,5 g Y(NO₃)₃*5H₂O werden in 0,5 1 VE-Wasser gelöst. Daneben wird eine 20%ige Sodalösung (851 g) bereitgestellt. Nun werden bei 65°C unter gutem Rühren die Sodalösung und die Metallsalzlösung gleichzeitig zugetropft unter Einhaltung des pH-Wertes von 5,6. Danach wird die Fällung für eine Stunde unter Durchleiten von Luft bei 65°C gerührt. Schließlich wird durch Zugabe weiterer Sodalösung der pH-Wert auf 7,5 angehoben. Nach Abnutschen der Fällung wird der Filterkuchen mit 20 1 VE-Wasser gewaschen, über Nacht bei 120°C getrocknet und für 4 h bei 400°C calciniert. 25 g dieses Pulvers wurden mit 45 Nl/h Wasserstoff und 5 Nl/h Stickstoff nach folgendem Temperaturprogramm reduziert: Innerhalb einer Stunde auf 300°C aufheizen und 4 Stunden halten, innerhalb einer Stunde aufheizen auf 470°C, 9 Stunden halten, innerhalb einer Stunde aufheizen auf 600°C, 3 Stunden halten und unter Stickstoff abkühlen lassen.
Die Analyse der reduzierten Stränge durch Röntgenbeugung ergab, dass mehr als 70% des Cobalts in reduzierter Form vorliegen. Die mittlere Co-Kristallitgröße beträgt 6,5 nm. Der Co-Gehalt beträgt 49%, der Ni-Gehalt 1% der Gesamtmasse.

### Beispiel 6

### Hydrierung von Dimethylaminopropionitril in Gegenwart des Katalysators aus Beispiel 5

In einen 270 ml-Druckautoklaven mit Begasungsrührer wurden 6 g des Katalysators aus Beispiel 5 mit 57,3 g Dimethylaminopropionitril eingebaut. Anschließend wurde der Autoklav mit Stickstoff inertisiert. Dann wurden 50 g Ammoniak aufgepreßt und auf 80°C erhitzt. Bei dieser Temperatur wurde Wasserstoff auf 80 bar aufgepreßt. Nach einer Reaktionszeit von 125 min wurde auf Raumtemperatur abgekühlt, entspannt und der ammoniakfreie Reaktoraustrag mittels Gaschromatographie untersucht. Der Umsatz an Dimethylaminopropionitril (DMAPN) betrug 100%, die Selektivität zu Dimethylaminopropylamin (DMAPA) betrug 98,4%.

### Vergleichsbeispiel 3

### Herstellung eines trägerfreien Katalysators analog Beispiel 1 von WO 97/10052

In einem Rührkolben wird eine Lösung aus 200 ml VE-Wasser und 42,4 g Na₂CO₃ vorgelegt und auf 80°C erwärmt. Zu dieser Lösung wird eine zweite Lösung, bestehend aus 200 ml VE-Wasser, 66,14 g Co(NO₃)₂*6H₂O und 30,62 g Cr(NO₃)₃*9H₂O innerhalb weniger Minuten unter Rühren zugetropft. Das molare Verhältnis zu Co:Cr beträgt 3:1. Nach dem Zutropfen wird noch 20 min bei 80°C gerührt, danach wird der Niederschlag abfiltriert und dieser mit 1,5 1 80°C warmem Wasser gewaschen. Der gewaschene Filterkuchen wird bei 120°C für 12 h getrocknet und anschließend zur Carbonatzersetzung bei 300°C für 3 h calciniert. Der oxidische Katalysator wird danach mit Wasserstoff 29 h lang bei 350°C reduziert.

### Vergleichsbeispiel 4

### Hydrierung von Dimethylaminopropionitril in Gegenwart des Katalysators aus Vergleichsbeispiel 3

In einen 270 ml-Druckautoklaven mit Begasungsrührer wurden 6 g des Katalysators aus Vergleichsbeispiel 3 mit 57,3 g Dimethylaminopropionitril eingebaut. Anschließend wurde der Autoklav mit Stickstoff inertisiert. Dann wurden 50 g Ammoniak aufgepreßt und auf 80°C erhitzt. Bei dieser Temperatur wurde Wasserstoff auf 80 bar aufgepreßt. Nach einer Reaktionszeit von 333 min wurde auf Raumtemperatur abgekühlt, entspannt und der ammoniakfreie Reaktoraustrag mittels Gaschromatographie untersucht. Der Umsatz an DMAPN betrugt 22,6%, die Selektivität zu DMAPA betrug 83,6%.

Dieses Vergleichsbeispiel macht deutlich, dass ein trägerfreier Katalysator, der gemäß Beispiel 1 von WO 97/10052 präpariert wurde, den erfindungsgemäßen Katalysatoren in Hinsicht Aktivität und Selektivität unterlegen ist.

## Patentansprüche

1. Verfahren zur Herstellung primärer Amine durch Hydrierung von Nitrilen in Anwesenheit eines Katalysators enthaltend Cobalt und gegebenenfalls zusätzlich Nickel sowie mindestens ein weiteres Dotiermetall auf einem teilchenförmigen Trägermaterial, **dadurch gekennzeichnet, daß** das Cobalt und gegebenenfalls das Nickel eine mittlere Teilchengröße von 3 bis 30 nm im aktiven Katalysator aufweisen.

2. Verfahren nach Anspruch 1, **dadurch gekennzeichnet, daß** der Katalysator gemäß den folgenden Schritten hergestellt wird:
a) gemeinsame Fällung von Cobalt und gegebenenfalls zusätzlich Nickel sowie mindestens einem weiteren Dotiermetall aus einer Lösung, die entsprechende Salze der genannten Metalle enthält, auf ein teilchenförmiges Trägermaterial,
b) anschließende Trocknung und/oder Calcinierung,
c) gegebenenfalls Verformung; und
d) Reduktion.

3. Verfahren nach Anspruch 1 oder 2, **dadurch gekennzeichnet, daß** das mindestens eine weitere Dotiermetall ausgewählt ist aus Metallen der Gruppen III B, IV B, V B, VI B, VII B, II A, III A und VI A und der Gruppe der Lanthaniden des Periodensystems der Elemente.

4. Verfahren nach einem der Ansprüche 1 bis 3, **dadurch gekennzeichnet, daß** der Katalysator mindestens ein weiteres Dotiermetall ausgewählt aus Silber, Gold und Ruthenium enthält.

5. Verfahren nach einem der Ansprüche 1 bis 4, **dadurch gekennzeichnet, daß** das Massenverhältnis von Cobalt und gegebenenfalls Nickel zu der Summe aus Trägermaterial und mitgefälltem Dotiermetall in Form von Oxid im Bereich von 20:80 bis 80:20 liegt und/oder das Massenverhältnis von Trägermaterial zu dem mitgefällten Dotiermetall in Form von Oxid im Bereich von 98:2 bis 30:70 liegt.

6. Verfahren nach einem der Ansprüche 1 bis 5, **dadurch gekennzeichnet, daß** mindestens 65% des Cobalt und gegebenenfalls Nickel in reduzierter Form vorliegen.

7. Verfahren nach einem der Ansprüche 1 bis 6, **dadurch gekennzeichnet, daß** die Reduktion in Schritt d) bei einem Verhältnis von Wasserstoffmenge zur Katalysatormenge von > als 1 Nm³/h x kg_{Kat.} durchgeführt wird.

8. Verfahren nach einem der Ansprüche 1 bis 7, **dadurch gekennzeichnet, daß** Nitrile der folgenden allgemeinen Formeln (I), (II), (III) oder (IV) eingesetzt werden:
R¹₃₋ₙX-A-CN (I)
worin die Symbole die folgenden Bedeutungen haben:
X O, S, N, P oder C,
A -(CR²R³)ₘ-, worin R² und R³ unabhängig voneinander substituierte oder unsubstituierte Aryl-, Alkyl- (verzweigt oder unverzweigt), Cykloalkylreste oder Wasserstoff bedeuten und m 0 bis 8 ist, oder
eine C₂- bis C₈-Alkylenkette, wobei die Alkylenkette durch 1 bis 4 Heteroatome, die nicht benachbart sind, unterbrochen ist, oder
Phenylen, Cyclohexylen, gegebenenfalls substituiert mit substituierten oder unsubstituierten Aryl-, Alkyl- (verzweigt oder unverzweigt), Cykloalkylresten oder mit Heteroatome, bevorzugt Sauerstoff oder Stickstoff, enthaltenden Resten;
n 2 (im Falle von X = O, S), 1 (im Falle von X = N, P) und 0 (im Falle von X = C);
R¹ substituierte oder unsubstituierte Aryl-, Alkyl- (verzweigt oder unverzweigt), Cykloalkylreste oder Wasserstoff, wobei im Falle von m = 0 oder 1 R¹ auch unterschiedliche Reste aus der für R¹ genannten Gruppe sein können oder zwei Reste R¹ gemeinsam mit dem Heteroatom X (N, P, C) einen cyclischen Rest bilden können, der gegebenenfalls beliebig substituiert sein kann, wobei der Rest aromatisch oder cykloaliphatisch sein kann;
NC-A-CN (II)
worin A die obenstehende Bedeutung hat; 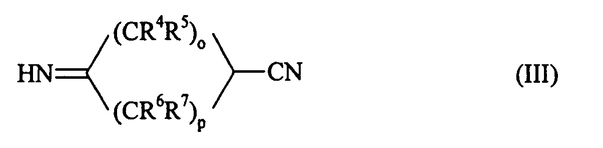 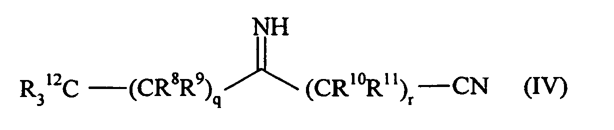 worin R⁴, R⁵, R⁶, R⁷, R⁸, R⁹, R¹⁰, R¹¹ und R¹² unabhängig voneinander substituierte oder unsubstituierte Aryl-, Alkyl- (verzweigt oder unverzweigt), Cykloalkylreste oder Wasserstoff bedeuten,
und
o, p, q, r 0, 1, 2, 3, 4 oder 5 bedeuten.

9. Verfahren nach Anspruch 8, **dadurch gekennzeichnet, daß** die folgenden Nitrile eingesetzt werden:
R₃₋ₙX-(CR²R³)ₘ -CN (Ia)
worin
X O oder N ist,
m 1 oder 2 ist; oder
NC-(CR²R³)ₘ-CN (IIa)
worin R² und R³ unabhängig voneinander Alkylreste (verzweigt oder unverzweigt) oder Wasserstoff bedeuten und m 2 bis 4 ist; oder
Iminonitrile der allgemeinen Formeln III oder IV.

10. Verwendung eines Katalysators gemäß einem der Ansprüche 1 bis 7 in einem Verfahren zur Herstellung primärer Amine durch Hydrierung von Nitrilen.

## Claims

1. A process for preparing primary amines by hydrogenating nitriles in the presence of a catalyst comprising cobalt and if appropriate, in addition, nickel and also at least one further doping metal on a particulate support material, wherein the cobalt and, if appropriate, the nickel have an average particle size of from 3 to 30 nm in the active catalyst.

2. The process according to claim 1, wherein the catalyst is prepared by the following steps:
a) coprecipitation of cobalt and if appropriate, in addition, nickel and also at least one further doping metal from a solution comprising the corresponding salts of the metals mentioned onto a particulate support material,
b) subsequent drying and/or calcining,
c) if appropriate, molding; and
d) reduction.

3. The process according to claim 1 or 2, wherein the at least one further doping metal is selected from the group consisting of metals of groups IIIB, IVB, VB, VIB, VIIB, IIA, IIIA and VIA and the lanthanide group of the periodic table.

4. The process according to any of claims 1 to 3, wherein the catalyst comprises at least one further doping metal selected from the group consisting of silver, gold and ruthenium.

5. The process according to any of claims 1 to 4, wherein the mass ratio of cobalt and, if appropriate, nickel to the sum of support material and coprecipitated doping metal in oxide form is in the range from 20:80 to 80:20 and/or the mass ratio of support material to cbncomitantly precipitated doping metal in oxide form is in the range from 98:2 to 30:70.

6. The process according to any of claims 1 to 5, wherein at least 65% of the cobalt and, if appropriate, the nickel are present in reduced form.

7. The process according to any of claims 1 to 6, wherein the reduction in step d) is carried out at a ratio of hydrogen quantity to catalyst quantity of > 1 Nm³/h x kg_{cat}.

8. The process according to any of claims 1 to 7, wherein nitriles of the following general formulae (I), (II), (III) or (IV) are used:
R¹₃₋ₙX-A-CN (I)
where the symbols are defined as follows:
X is O, S, N, P or C,
A is -(CR²R³)ₘ-, where R² and R³ are each independently substituted or unsubstituted aryl, alkyl (branched or unbranched) or cycloalkyl radicals or hydrogen and m is from 0 to 8, or
is a C₂- to C₈-alkylene chain, where the alkylene chain is interrupted by from 1 to 4 nonneighboring heteroatoms, or
is phenylene, cyclohexylene, optionally substituted by radicals comprising substituted or unsubstituted aryl, alkyl (branched or unbranched) or cycloalkyl radicals or by heteroatoms, preferably oxygen or nitrogen;
n is 2 (when X = O, S), 1 (when X = N, P) or 0 (when X = C);
R¹ is a substituted or unsubstituted aryl, alkyl (branched or unbranched) or cycloalkyl radical or hydrogen, although when m = 0 or 1, the R¹ radicals may also be differing radicals selected from the R¹ group, or two R¹ radicals together with the heteroatom X (N, P, C) may form a cyclic radical which may be substituted in any desired manner and may be aromatic or cycloaliphatic;
NC-A-CN (II)
where A is as defined above; 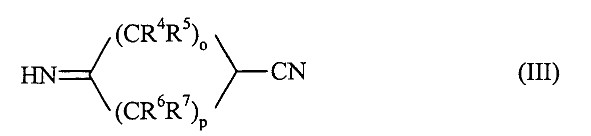 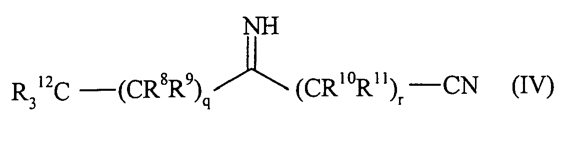 where R⁴, R⁵, R⁶, R⁷, R⁸, R⁹, R¹⁰, R¹¹ and R¹² are each independently substituted or unsubstituted aryl, alkyl (branched or unbranched) or cycloalkyl radicals or hydrogen,
and
o, p, q, r are 0, 1, 2, 3, 4 or 5.

9. The process according to claim 8, wherein the following nitriles are used:
R₃₋ₙX-(CR²R³)ₘ -CN (Ia)
where
X is O or N,
m is 1 or 2; or
NC-(CR²R³)ₘ-CN (IIa)
where R² and R³ are each independently alkyl radicals (branched or unbranched) or hydrogen and m is from 2 to 4; or
iminonitriles of the general formulae III or IV.

10. The use of a catalyst according to any of claims 1 to 7 in a process for preparing primary amines by hydrogenating nitriles.

## Revendications

1. Procédé de préparation d'amines primaires par hydrogénation de nitriles en présence d'un catalyseur renfermant du cobalt et éventuellement, en outre, du nickel, ainsi qu'au moins un métal de dopage supplémentaire sur une matière de support particulaire, **caractérisé en ce que** le cobalt et, éventuellement, le nickel présentent une taille de particules moyenne de 3 à 30 nm dans le catalyseur actif.

2. Procédé selon la revendication 1, **caractérisé en ce que** le catalyseur est préparé conformément aux étapes suivantes :
a) précipitation conjointe du cobalt et éventuellement, en outre, du nickel, ainsi que d'au moins un métal de dopage supplémentaire dans la solution, qui contient les sels correspondants des métaux mentionnés, sur une matière de support particulaire ;
b) puis, séchage et/ou calcination ;
c) éventuellement, moulage ; et
d) réduction.

3. Procédé selon la revendication 1 ou 2, **caractérisé en ce que** l'au moins un métal de dopage supplémentaire est choisi parmi des métaux des groupes III B, IV B, V B, VI B, VII B, II A, III A et VI A et du groupe des lanthanides du système périodique des éléments.

4. Procédé selon l'une quelconque des revendications 1 à 3, **caractérisé en ce que** le catalyseur renferme au moins un métal de dopage supplémentaire choisi parmi l'argent, l'or et le ruthénium.

5. Procédé selon l'une quelconque des revendications 1 à 4, **caractérisé en ce que** le rapport massique entre le cobalt et, éventuellement, le nickel, et la somme de la matière de support et du métal de dopage co-précipité sous la forme d'oxyde est dans la plage de 20:80 à 80:20 et/ou le rapport massique entre la matière de support et le métal de dopage co-précipité sous la forme d'oxyde est dans la plage de 98:2 à 30:70.

6. Procédé selon l'une quelconque des revendications 1 à 5, **caractérisé en ce qu'**au moins 65 % du cobalt et, éventuellement, le nickel sont présents sous une forme réduite.

7. Procédé selon l'une quelconque des revendications 1 à 6, **caractérisé en ce que** la réduction dans l'étape d) est réalisée à un rapport entre la quantité d'hydrogène et la quantité de catalyseur supérieur à 1 Nm³/h x kg_{cat}.

8. Procédé selon l'une quelconque des revendications 1 à 7, **caractérisé en ce que** l'on utilise des nitriles de formules générales (I), (II), (III) ou (IV) suivantes :
**R¹₃₋ₙX-A-CN** **(I)**
dans lesquelles les symboles présentent les significations suivantes :
X représente un atome d'oxygène, un atome de soufre, un atome d'azote, un atome de phosphore ou un atome de carbone ;
A représente un groupe -(CR²R³)ₘ-, dans lequel R² et R³ représentent, indépendamment l'un de l'autre, des radicaux aryle, alkyle (ramifiés ou non ramifiés), cycloalkyle, substitués ou non substitués, ou un atome d'hydrogène, et m vaut de 0 à 8, ou
une chaîne alkylène en C₂ à C₈, où la chaîne alkylène est interrompue par de 1 à 4 hétéro-atomes qui ne sont pas voisins, ou un groupe phénylène, un groupe cyclohexylène, éventuellement substitués par des radicaux aryle, alkyle (ramifiés ou non ramifiés), cycloalkyle, substitués ou non substitués, ou par des radicaux renfermant des hétéroatomes, de préférence, un atome d'oxygène ou un atome d'azote ;
n vaut 2 (au cas où X = O, S), 1 (au cas où X = N, P) et 0 (au cas où X = C) ;
R¹ représente des radicaux aryle, alkyle (ramifiés ou non ramifiés), cycloalkyle, substitués ou non substitués, ou un atome d'hydrogène, où, au cas où m = 0 ou 1, les groupes R¹ peuvent également être des radicaux différents parmi le groupe mentionné pour R¹, ou deux radicaux R¹, conjointement avec l'hétéroatome X (N, P, C) peuvent former un radical cyclique qui peut éventuellement être substitué, de façon quelconque, où le radical peut être aromatique ou cycloaliphatique ;
**NC-A-CN** **(II)**
dans laquelle A présente la signification ci-dessus ; 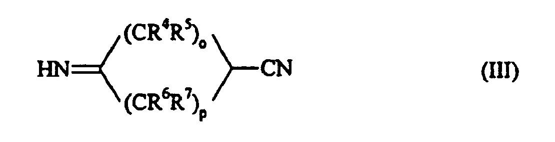 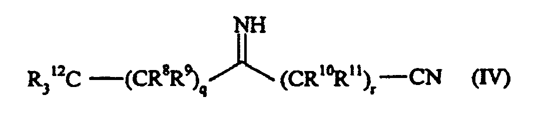 dans lesquelles R⁴, R⁵, R⁶, R⁷, R⁸, R⁹, R¹⁰, R¹¹ et R¹² représentent, indépendamment les uns des autres, des radicaux aryle, alkyle (ramifiés ou non ramifiés), cycloalkyle, substitués ou non substitués, ou un atome d'hydrogène,
et
o, p, q, r valent 0, 1, 2, 3, 4 ou 5.

9. Procédé selon la revendication 8, **caractérisé en ce que** l'on utilise les nitriles suivants :
**R₃₋ₙX-(CR²R³)ₘ-CN** **(Ia)**
dans lequel
X représente un atome d'oxygène ou un atome d'azote ;
m vaut 1 ou 2 ; ou
**NC-(CR²R³)ₘ-CN** **(IIa)**
dans lequel R² et R³ représentent, indépendamment l'un de l'autre, des radicaux alkyle (ramifiés ou non ramifiés) ou un atome d'hydrogène, et m vaut de 2 à 4 ; ou
des iminonitriles des formules générales III ou IV.

10. Utilisation d'un catalyseur selon l'une quelconque des revendications 1 à 7 dans un procédé de préparation d'amines primaires par hydrogénation de nitriles.
